Europäisches Patentamt

European Patent Office

Office européen des brevets

(19)

(11) Veröffentlichungsnummer: **0 355 675**
**A1**

(12) **EUROPÄISCHE PATENTANMELDUNG**

(21) Anmeldenummer: 89115062.5

(22) Anmeldetag: 16.08.89

(51) Int. Cl.⁴: **C07C 309/70 , C07C 303/26 ,
C11D 1/28**

(30) Priorität: 25.08.88 DE 3828892

(43) Veröffentlichungstag der Anmeldung:
28.02.90 Patentblatt 90/09

(84) Benannte Vertragsstaaten:
ES

(71) Anmelder: Henkel Kommanditgesellschaft auf
Aktien
Postfach 1100 Henkelstrasse 67
D-4000 Düsseldorf 1(DE)

(72) Erfinder: Behler, Ansgar, Dr.
Siegfriedstrasse 80
D-4250 Bottrop(DE)
Erfinder: Eierdanz, Horst, Dr.
Am Eichelkamp 116
D-4010 Hilden(DE)

(54) Ölsäure-niedrigalkylestersulfonate, Verfahren zu ihrer Herstellung und ihre Verwendung.

(57) Ölsäure-niedrigalkylestersulfonate, erhältlich durch Sulfonierung technischer Oleatschnitte der Zusammensetzung (jeweils als Niedrigalkylestern)

| | |
|---|---|
| Ölsäure | 78 - 92 Gew.-Teile |
| Linolsäure | 2 - 10 Gew.-Teile |
| Palmitinsäure | 2 - 5 Gew.-Teile |
| Stearinsäure | 2 - 7 Gew.-Teile |

sowie gegebenenfalls jeweils 1 Gew.-Teil nicht übersteigende Mengen an anderen gesättigten oder ungesättigten Fettsäure-niedrigalkylestern mit 12 bis 22 Kohlenstoffatomen im Fettsäureteil mit gasförmigem Schwefeltrioxid in Einsatzverhältnissen von 1,0 bis 1,5 Mol Schwefeltrioxid pro Mol der in den Fettsäureestern vorhandenen olefinischen Doppelbindungen bei Temperaturen von 15 bis 90°C sowie Neutralisation und Hydrolyse des erhaltenen Sulfonierungsproduktes mit wässrigen Basen, weisen überraschend hohe Sulfiergrade von mindestens 80 % auf.

EP 0 355 675 A1

## Ölsäure-niedrigalkylestersulfonate, Verfahren zu ihrer Herstellung und ihre Verwendung.

Die Erfindung betrifft Ölsäure-niedrigalkylestersulfonate mit hohen Sulfiergraden.

Die Herstellung von bekannten Ölsäure-niedrigalkylestersulfonaten erfolgt durch Umsetzung von Ölsäurealkylestern mit Sulfonierungsmitteln wie Schwefeltrioxid oder Oleum. So beschreiben die EP-A 0 130 753 sowie die DE-A 3 606 868 die Sulfonierung von ungesättigten Fettsäure-niedrigalkylestern mit gasförmigem, luftverdünntem Schwefeltrioxid. Üblicherweise werden hierzu keine hochreinen Ölsäureester, sondern technische Gemische derselben eingesetzt, die aus natürlichen Fetten und Ölen tierischer oder pflanzlicher Herkunft gewonnen werden. Diese technischen Ölsäureester enthalten neben weiteren ungesättigten Fettsäureestern im allgemeinen auch Ester gesättigter Fettsäuren.

Bei der Sulfonierung von technischen Gemischen aus ungesättigten und gesättigten Fettsäureestern reagieren selektiv nur die ungesättigten Ester mit Schwefeltrioxid; die gesättigten Ester verbleiben unverändert im Sulfonierungsgemisch. Die Höhe des Sulfonierungsgrades sollte also abhängig von dem Gehalt an ungesättigten Fettsäureestern in dem technischen Estergemisch sein. Ein Maß für diesen Gehalt ist die Jodzahl des technischen Estergemisches. Technische Ölsäureester unterschiedlicher Herkunft mit gleicher Jodzahl sollten also unter gleichen Reaktionsbedingungen die gleichen Sulfiergrade ergeben.

Überraschenderweise wurde nun gefunden, daß Ölsäure-niedrigalkylester, insbesondere Ölsäuremethylester der Zusammensetzung (jeweils als Niedrigalkylester)

| Ölsäure | 78 - 92 Gew.-Teile |
| Linolsäure | 2 - 10 Gew.-Teile |
| Palmitinsäure | 2 - 5 Gew.-Teile |
| Stearinsäure | 2 - 7 Gew.-Teile |

sowie gegebenenfalls jeweils 1 Gew.-Teil nicht übersteigende Mengen an anderen gesättigten oder ungesättigten Fettsäure-niedrigalkylestern mit 12 bis 22 Kohlenstoffatomen im Fettsäureteil, die aus hochölsäurehaltigem Sonnenblumenöl erhältlich sind, bei der Sulfonierung deutlich höhere Sulfiergrade ergeben als z.B. technische Ölsäuremethylester auf Basis von Rindertalg (Ölsäuregehalt 63-73 %) oder auf Basis hochreiner Ölsäure (99 %-ig), obwohl die Jodzahlen dieser Ölsäuremethylester praktisch identisch sind.

Demgemäß betrifft die Erfindung Ölsäure-niedrigalkylestersulfonate, erhältlich durch Sulfonierung technischer Oleatschnitte der Zusammensetzung (jeweils als Niedrigalkylester)

| Ölsäure | 78 - 92 Gew.-Teile |
| Linolsäure | 2 - 10 Gew.-Teile |
| Palmitinsäure | 2 - 5 Gew.-Teile |
| Stearinsäure | 2 - 7 Gew.-Teile |

sowie gegebenenfalls jeweils 1 Gewichtsteil nicht übersteigende Mengen an anderen gesättigten oder ungesättigten Fettsäure-niedrigalkylestern mit 12 bis 22 Kohlenstoffatomen im Fettsäureteil mit gasförmigem Schwefeltrioxid in Einsatzverhältnissen von 1,0 bis 1,5 Mol Schwefeltrioxid pro Mol der in den Fettsäureestern vorhandenen olefinischen Doppelbindungen bei Temperaturen von 15 bis 90°C sowie Neutralisation und Hydrolyse des erhaltenen Sulfonierungsproduktes mit wässrigen Basen. Unter Niedrigalkylestern im Sinne der Erfindung sind Ester zu verstehen, die 1 bis 6, insbesondere 1 bis 4 Kohlenstoffatome im Alkanolrest enthalten; Methylester sind bevorzugt.

Gemäß einer bevorzugten Ausführungsform der Erfindung weisen die Ölsäure-niedrigalkylestersulfonate Sulfiergrade von mindestens 80 % auf.

Die Sulfonierung der eingesetzten technischen Oleatschnitte mit gasförmigem Schwefeltrioxid kann in üblichen Sulfonierungsreaktoren, insbesondere vom Typ der Fallfilmreaktoren, erfolgen. Dabei wird Schwefeltrioxid mit Luft oder Stickstoff verdünnt eingesetzt, vorzugsweise in Form eines Gasgemisches mit ca. 1 bis 10 Vol.-% Schwefeltrioxid. Ein besonders bevorzugter Temperaturbereich für die Sulfonierung beträgt 15 bis 30°C. Weiterhin ist besonders bevorzugt, die Sulfonierung mit gasförmigem Schwefeltrioxid in Einsatzverhältnissen von 1,0 bis 1,2 Mol Schwefeltrioxid pro Mol olefinischer Doppelbindungen vorzunehmen.

Als für die Neutralisation bzw. Hydrolyse geeignete Basen eignen sich Oxide bzw. Hydroxide von Alkalimetallen wie Natrium, Kalium und Lithium oder von Erdalkalimetallen wie Magnesium oder Calcium oder organische Basen wie Ammoniak, Ethanolamin, Diethanolamin oder Triethanolamin, jeweils in wässriger Lösung. Die Verwendung von wässriger Natriumlauge ist bevorzugt.

Zweckmäßigerweise wird das erhaltene Sulfonierungsprodukt unmittelbar der Neutralisation und Hydrolyse zugeführt, d.h. es ist - anders als bei üblichen Sulfonierungsverfahren für Ester gesättigter Fettsäuren - eine Nachreaktionszeit des Sulfonierungsproduktes vor der Hydrolyse und Neutralisation nicht vorgesehen.

Gemäß einer weiteren bevorzugten Ausführungsform der Erfindung wird das Sulfonierungsprodukt neutralisiert und unter Zufuhr von wässrigen Basen zur Einhaltung eines pH-Wertes von 6 bis 8, insbesondere von 6,5 bis 7,5, durch 2- bis 5-stündiges Erhitzen bei mindestens 70 °C hydrolysiert. Die obere Grenze der Hydrolysetemperatur ist durch den Siedepunkt des Wassers bestimmt; man kann jedoch auch die Hydrolyse in Druckreaktoren bei Temperaturen von mehr als 100 °C durchführen. Zweckmäßigerweise arbeitet man jedoch drucklos bei Hydrolysetemperaturen von etwa 90 °C.

Die Einhaltung des hier angegebenen pH-Wertes ist wichtig; erfolgt z.B. lediglich die Neutralisation ohne weitere Basenzufuhr während der Hydrolyse, sinkt der pH-Wert des Reaktionssystems infolge von Umlagerungsreaktionen der Sulfonierungsprodukte rasch unter 6, wodurch die Verseifung der Esterfunktionen gefördert wird. Der gleiche Effekt tritt ein, wenn der pH-Wert des Reaktionssystems für längere Zeit größer als 8 wird.

Die erfindungsgemäßen Ölsäure-niedrigalkylestersulfonate können kontinuierlich oder diskontinuierlich hergestellt werden. Bei der diskontinuierlichen Herstellung kann man das Sulfonierungsprodukt in eine vorgelegte wässrige Lösung der Base geben, wobei das molare Verhältnis von Base zu Schwefeltrioxid bzw. Sulfongruppen in dem Sulfonierungsprodukt mehr als 1 : 1 bis zu 1,3 : 1, insbesondere 1,1 - 1,3 : 1, beträgt.

Zwar erfolgt bei dieser Variante die Hydrolyse zunächst bei pH-Werten von mehr als 8, dieser an sich ungünstige pH-Bereich wird jedoch bei dieser Verfahrensführung rasch unterschritten.

Die Erfindung betrifft weiterhin Verfahren zur Herstellung von Ölsäure-niedrigalkylestersulfonaten gemäß den obigen Merkmalen. Schließlich betrifft die Erfindung die Verwendung der Ölsäure-niedrigalkylestersulfonate mit den obengenannten Merkmalen einerseits als oberflächenaktive Mittel und andererseits zur Herstellung von Ölsäure-niedrigalkylestersulfonaten mit Sulfiergraden von mindestens 80 %.

Die Erfindung wird im folgenden anhand von Ausführungsbeispielen und Vergleichsversuchen näher erläutert.

Als Ausgangsmaterial wurden technische Ölsäuremethylester der in Tabelle 1 angegebenen gaschromatographisch ermittelten Zusammensetzung (in Flächenprozent) und ein hochreiner Ölsäuremethylester (99 %-ig) eingesetzt; in der Tabelle und im folgenden wird mit MeSBN ein technischer Ölsäuremethylester bezeichnet, der aus einer Sonnenblumensorte gemäß US-C 4 627 192 stammt. Mit MeTiO$_5$ wird ein handelsüblicher technischer Ölsäuremethylester aus Rindertalg bezeichnet.

3

Tabelle 1

| Zusammensetzung der untersuchten technischen Ölsäuremethylester | | |
|---|---|---|
| | MeSBN | MeTiO$_5$ |
| C$_{12}$ gesättigt | - | 0,63 % |
| C$_{14}$ gesättigt | < 0,5 % | 2,74 % |
| C$_{15}$ gesättigt | - | 0,21 % |
| C$_{16}$ gesättigt | 3,5 % | 4,21 % |
| C$_{17}$ gesättigt | - | < 1 % |
| C$_{18}$ gesättigt | 3,6 % | 1,20 % |
| C$_{20}$ gesättigt | 0,3 % | < 1 % |
| C$_{22}$ gesättigt | < 1 % | < 1 % |
| C$_{16}$ einfach ungesättigt | < 1 % | 5,01 % |
| C$_{18}$ einfach ungesättigt | 82,8 % | 67,96 % |
| C$_{18}$ zweifach ungesättigt | 8,4 % | 7,54 % |
| C$_{18}$ dreifach ungesättigt | < 1 % | 0,72 % |
| C$_{20}$ einfach ungesättigt | < 1 % | 1,01 % |

Die Kennzahlen (Jod- und Verseifungszahl) der untersuchten Ölsäuremethylester sind in Tabelle 2 zusammengefaßt. Mit MeNOF ist der 99 %-ige Ölsäuremethylester bezeichnet.

Tabelle 2

| Kennzahlen der untersuchten Ölsäuremethylester | | |
|---|---|---|
| Ölsäuremethylester | Jodzahl | Verseifungszahl |
| MeSBN | 85 | 188,8 |
| MeTiO$_5$ | 86,7 | 191,6 |
| MeNOF | 85,2 | 188,3 |

Die Sulfonierung erfolgte unter gleichen Bedingungen in einem Labor-Standreaktor mit Mantelkühlung unter den weiter unten erläuterten Bedingungen. Als Sulfonierungsmittel diente gasförmiges Schwefeltrioxid, erzeugt durch Erhitzen von Oleum und verdünnt mit Stickstoff (5 Vol.-% Schwefeltrioxidgehalt im Stickstoffstrom).

Nach Beendigung der Reaktion wurde das Reaktionsgemisch in ein Becherglas überführt, das mit der Elektrode eines pH-Meters und einem Rührer ausgerüstet war. Das anfallende Reaktionsprodukt wurde durch Zugabe von 25 gew.-%-iger wäßriger Natriumhydroxidlösung sofort auf einen pH-Wert von 7 ± 0,5 neutralisiert.

Zur Hydrolyse wurde das neutralisierte Reaktionsprodukt anteilweise 6 Stunden lang auf 90° C erhitzt. Durch Zugabe von 25 gew.-%-iger Natriumhydroxidlösung wurde dabei der pH-Wert der Lösung ständig auf 7 ± 0,5 gehalten.

Die verschiedenen Ölsäuremethylester wurden wie oben beschrieben unter den in der folgenden Tabelle 3 angegebenen Bedingungen bezüglich Reaktionstemperatur und molaren Verhältnissen von Schwefeltrioxid zu ungesättigten Doppelbindungen umgesetzt; die nach der Neutralisation und Hydrolyse ermittelten Sulfiergrade sind ebenfalls in der Tabelle 3 zusammengefaßt.

4

Tabelle 3

| Reaktionsbedingungen und Sulfiergrade | | | |
|---|---|---|---|
| | Temp. (°C) | $SO_3$: C=C | Sulfiergrad (%) |
| MeSBN | 15 | 1,0:1 | 80 |
| MeTiO₅ | 15 | 1,0:1 | 74 |
| MeNOF | 15 | 1,0:1 | 65 |
| MeSBN | 15 | 1,2:1 | 84 |
| MeTiO₅ | 15 | 1,2:1 | 76 |
| MeNOF | 15 | 1,2:1 | 78 |
| MeSBN | 30 | 1,0:1 | 76 |
| MeTiO₅ | 30 | 1,0:1 | 72 |
| MeNOF | 30 | 1,0:1 | 58 |
| MeSBN | 30 | 1,2:1 | 81 |
| MeTiO₅ | 30 | 1,2:1 | 78 |
| MeNOF | 30 | 1,2:1 | 78 |

In allen untersuchten Fällen ergaben sich bei Einsatz des Ölsäuremethylesters gemäß der Erfindung MeSBN die höchsten Sulfiergrade, obwohl die Methylester auf Basis der anderen Rohstoffe, d.h. Rindertalg (MeTiO₅) und hochreiner Ölsäure (MeNOF) die gleiche Jodzahl aufwiesen.

**Ansprüche**

1. Ölsäure-niedrigalkylestersulfonate, erhältlich durch Sulfonierung technischer Oleatschnitte der Zusammensetzung (jeweils als Niedrigalkylester)

| Ölsäure | 78 - 92 Gew.-Teile |
|---|---|
| Linolsäure | 2 - 10 Gew.-Teile |
| Palmitinsäure | 2 - 5 Gew.-Teile |
| Stearinsäure | 2 - 7 Gew.-Teile |

sowie gegebenenfalls jeweils 1 Gew.-Teil nicht übersteigende Mengen an anderen gesättigten oder ungesättigten Fettsäure-niedrigalkylestern mit 12 bis 22 Kohlenstoffatomen im Fettsäureteil mit gasförmigem Schwefeltrioxid in Einsatzverhältnissen von 1,0 bis 1,5 Mol Schwefeltrioxid pro Mol der in den Fettsäureestern vorhandenen olefinischen Doppelbindungen bei Temperaturen von 15 bis 90° C sowie Neutralisation und Hydrolyse des erhaltenen Sulfonierungsproduktes mit wässrigen Basen.

2. Ölsäure-niedrigalkylestersulfonate nach Anspruch 1, gekennzeichnet durch Sulfiergrade von mindestens 80 %.

3. Ölsäure-niedrigalkylestersulfonate nach Anspruch 1 oder 2, erhältlich durch Sulfonierung mit gasförmigem Schwefeltrioxid bei Temperaturen von 15 bis 30° C.

4. Ölsäure-niedrigalkylestersulfonate nach mindestens einem der Ansprüche 1 bis 3, erhältlich durch Sulfonierung mit gasförmigem Schwefeltrioxid in Einsatzverhältnissen von 1,0 bis 1,2 Mol Schwefeltrioxid pro Mol olefinischer Doppelbindungen.

5. Ölsäure-niedrigalkylestersulfonate nach mindestens einem der Ansprüche 1 bis 4, gekennzeichnet durch eine Hydrolyse durch 2 bis 5-stündiges Erhitzen des mit wässrigen Basen neutralisierten Sulfonierungsproduktes auf Temperaturen von mindestens 70° C unter ständiger Zudosierung wässriger Basen zur Einhaltung eines pH-Wertes von 6 bis 8, insbesondere 6,5 bis 7,5.

6. Ölsäure-niedrigalkylestersulfonate nach mindestens einem der Ansprüche 1 bis 5, dadurch gekennzeichnet, daß man als wässrige Base Natronlauge verwendet.

7. Verfahren zur Herstellung von Ölsäure-niedrigalkylestersulfonaten, gekennzeichnet durch die Sulfonierung von technischen Oleatschnitten der Zusammensetzung (jeweils als Niedrigalkylester)

| Ölsäure | 78 - 92 Gew.-Teile |
|---|---|
| Linolsäure | 2 - 10 Gew.-Teile |
| Palmitinsäure | 2 - 5 Gew.-Teile |
| Stearinsäure | 2 - 7 Gew.-Teile |

sowie gegebenenfalls jeweils 1 Gew.-Teil nicht übersteigende Mengen an anderen gesättigten oder ungesättigten Fettsäure-niedrigalkylestern mit 12 bis 22 Kohlenstoffatomen im Fettsäureteil mit gasförmigem Schwefeltrioxid in Einsatzverhältnissen von 1,0 bis 1,5 Mol Schwefeltrioxid pro Mol der in den Fettsäureestern vorhandenen olefinischen Doppelbindungen bei Temperaturen von 15 bis 90°C sowie Neutralisation und Hydrolyse des erhaltenen Sulfonierungsproduktes mit wässrigen Basen.

8. Verfahren nach Anspruch 7, dadurch gekennzeichnet, daß die Ölsäure-niedrigalkylestersulfonate Sulfiergrade von mindestens 80 % aufweisen.

9. Verfahren nach Anspruch 7 oder 8, dadurch gekenzeichnet, daß die Sulfonierung mit gasförmigem Schwefeltrioxid bei Temperaturen von 15 bis 30°C erfolgt.

10. Verfahren nach mindestens einem der Ansprüche 7 bis 9, dadurch gekennzeichnet, daß die Sulfonierung mit gasförmigem Schwefeltrioxid in Einsatzverhältnissen von 1,0 bis 1,2 Mol Schwefeltrioxid pro Mol olefinischer Doppelbindungen erfolgt.

11. Verfahren nach mindestens einem der Ansprüche 7 bis 10, dadurch gekennzeichnet, daß die Hydrolyse durch 2 bis 5-stündiges Erhitzen des mit wässrigen Basen neutralisierten Sulfonierungsproduktes auf Temperaturen von mindestens 70°C unter ständiger Zudosierung wässriger Basen zur Einhaltung eines pH-Wertes von 6 bis 8, insbesondere 6,5 bis 7,5, erfolgt.

12. Verfahren nach mindestens einem der Ansprüche 7 bis 11, dadurch gekennzeichnet, daß man als wässrige Base Natronlauge verwendet.

13. Verwendung der Ölsäure-niedrigalkylestersulfonate nach mindestens einem der Ansprüche 1 bis 6 als oberflächenaktive Mittel.

14. Verwendung von technischen Oleatschnitten der Zusammensetzung (jeweils als Niedrigalkylester)

| Ölsäure· | 78 - 92 Gew.-Teile |
|---|---|
| Linolsäure | 2 - 10 Gew.-Teile |
| Palmitinsäure | 2 - 5 Gew.-Teile |
| Stearinsäure | 2 - 7 Gew.-Teile |

sowie gegebenenfalls 1 Gew.-Teil nicht übersteigende Mengen an anderen gesättigten oder ungesättigten Fettsäure-niedrigalkylestern mit 12 bis 22 Kohlenstoffatomen im Fettsäureteil zur Herstellung von Ölsäure-niedrigalkylestersulfonaten mit Sulfiergraden von mindestens 80 %.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 130 753 (LION CORP.) <br> * Seiten 3-6; Seite 8, Beispiel 1; Ansprüche * <br> --- | 1,7-13 | C 07 C 309/70 <br> C 07 C 303/26 <br> C 11 D 1/28 |
| A | GB-A-2 174 089 (LION CORP.) <br> * Seiten 4-10 *; & DE - A - 36 06868 (Kat. D) <br> --- | 1,7,13 | |
| A | GB-A-2 168 072 (LION CORP.) <br> * Seite 2, Beispiel 1; Ansprüche * <br> --- | 1,7,13 | |
| A | US-A-2 743 288 (W. H. C. RUEGGEBERG et al.) <br> * Spalte 11; Spalte 12, Beispiel 6 * <br> --- | 1,7,13 | |
| A | KARL F. MATTIL et al.: "BAILEY'S INDUSTRIAL OIL AND FAT PRODUCTS" <br> 3. Auflage, Verleger Daniel Swern, Interscience Publishers, a Division of John Wiley & Sons * Seiten 207,208 * <br> --- | 1 | |
| A,P | FOOD TECHNOLOGY <br> Juli 1989, Seiten 66-74; "Fats, Oils, and Fat Substitutes" * Seite 70, Spalte 2 * <br> ----- | 1 | RECHERCHIERTE SACHGEBIETE (Int. Cl.5) <br><br> C 07 C 309/00 <br> C 07 C 303/00 <br> C 11 D 1/00 |

Der vorliegende Recherchenbericht wurde für alle Patentansprüche erstellt

| Recherchenort | Abschlußdatum der Recherche | Prüfer |
|---|---|---|
| BERLIN | 30-10-1989 | RUFET J.M.A. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................................................
& : Mitglied der gleichen Patentfamilie, übereinstimmendes Dokument